# EUROPÄISCHE PATENTANMELDUNG

(11) **EP 2 055 255 A1**
(43) Veröffentlichungstag der Anmeldung: **06.05.2009**
(21) Anmeldenummer: 07119752.9
(22) Anmeldetag: 31.10.2007
(51) Int. Cl.: A61B 19/00, A61B 17/00

(54) **Verifizierung des Kalibrierungszustandes eines optischen Trackingsystems**

(71) Anmelder: BrainLAB AG, 85622 Feldkirchen (DE)
(72) Erfinder: Goldbach, Günter, 85457 Wörth/Wifling (DE)
(74) Vertreter: Schwabe - Sandmair - Marx

(57) **Zusammenfassung**

Die Erfindung betrifft ein Verfahren zur Verifizierung des Kalibrierungszustandes eines optischen Trackingsystems (1), bei dem
- ein Kalibrierungsgerät (20, 40), welches eine Trackingmarker-Konfiguration (27, 28; 41, 42) aufweist, in den Erfassungsbereich des Trackingsystems (1) gebracht wird;
- die Trackingmarker (27, 28; 41, 42) des Kalibrierungsgeräts (20, 40) vom Trackingsystem erfasst werden;
- die Anordnung der Trackingmarker (27, 28; 41, 42) verändert und/oder in einen Zustand gebracht wird, so dass das Trackingsystem (1) ein Erfassungsproblem erfährt;
- die Anordnung der Trackingmarker (27, 28; 41, 42) vermessen wird, bei der das Erfassungsproblem besteht bzw. ein Anordnungsbereich für die Trackingmarker (27, 28; 41, 42) vermessen wird, bei dem das Erfassungsproblem besteht; und bei dem
- wenn die vermessene Anordnung bzw. der Anordnungsbereich nicht einen vorbestimmten Wert hat oder nicht in einem vorbestimmten Wertebereich liegt, die Fehlkalibrierung des Trackingsystems (1) festgestellt wird.

Ferner betrifft sie eine Vorrichtung zur Verifizierung des Kalibrierungszustandes eines optischen Trackingsystems (1).

## Beschreibung

Die Erfindung betrifft die Verifizierung des Kalibrierungszustandes eines optischen Trackingsystems. Insbesondere findet die Erfindung ihre Anwendung im Bereich medizintechnisch genutzter bzw. medizintechnischer optischer Trackingsysteme, die dazu dienen, die Raumposition von Trackingmarkern (z.B. an medizinischen Instrumenten oder Apparaten bzw. an Patienten) zu ermitteln und medizintechnischen Navigationssystemen zur Verfügung zu stellen, um eine bildgeführte Chirurgie bzw. Behandlung durchführen zu können.

Bekannte auf dem Markt erhältliche Trackingsysteme solcher Art sind bei ihrer Lieferung vorkalibriert, d.h. die exakten Parameter der Anordnung ihrer Bauteile sind bekannt und werden bei der Ermittlung von Raumkoordinaten für Trackingmarker berücksichtigt. Ein generelles Problem solcher bei der Herstellung vorkalibrierter, stereoskopischer Trackingsysteme liegt in der Verifizierung der Stabilität der Kalibrierung über eine längere Benutzungszeitspanne ("im Feld"). Abgesehen von einigen sehr grundlegenden Funktionstest besteht bei bekannten Systemen keine Möglichkeit, die Genauigkeit des Systems zu überprüfen. Da medizinische Anwendungen solcher Trackingsystem aber immer häufiger Genauigkeitsanforderungen haben, die im Sub-Millimeterbereich liegen, wird die Verifizierung der Genauigkeit bzw. des Kalibrierungszustandes immer wichtiger.

Derzeit werden Trackingsysteme meist jährlich mit Hilfe eines großen Koordinaten-Vermessungssystems überprüft, das typischerweise auch dafür verwendet wird, solche Systeme zu kalibrieren. Dies kann jedoch nicht im Feld d.h. vor Ort bei dem Benutzer stattfinden, sondern die Trackingsysteme müssen zum Herstellern geliefert werden, wo ein solches Koordinaten-Vermessungssystem zur Verfügung steht. Dies ist mit hohem Aufwand und insbesondere hohen Kosten verbunden, und die Systeme sind während der entsprechenden Zeit nicht für den Benutzer verfügbar. Beim Hersteller oder bei der Stelle, wo die Kalibrierung durchgeführt werden kann, wird die getestete Vorrichtung anhand eines wesentlich besseren (typischerweise zehnmal besseren) Referenzstandards vermessen und eventuell neu kalibriert. Weil die Genauigkeit und die Stabilität der Trackingsysteme aber eine immer größere Bedeutung erlangen, die genannten Hersteller-Kalibrierungen oder Nach-Kalibrierungen teuer sind und einen Nutzungsausfall mit sich bringen, und weil nicht kalibrierte Systeme die Gefahr von Behandlungsfehlern mit sich bringen ist es äußerst wichtig, eine Verifizierung des Kalibrierungszustandes des optischen Trackingsystems vor Ort beim Benutzer und mit möglichst geringem Aufwand möglich zu machen.

Die vorliegende Erfindung hat sich dies zur Aufgabe gemacht.

Die genannte Aufgabe wird durch ein Verfahren zur Verifizierung des Kalibrierungszustandes eines optischen Trackingsystems gemäß dem Anspruch 1 sowie durch eine zu diesem Zweck bereitgestellte Vorrichtung gemäß dem Anspruch 12 gelöst. Die Unteransprüche definieren bevorzugte Ausführungsformen der Erfindung.

Bei dem erfindungsgemäßen Verfahren zur Verifizierung des Kalibrierungszustandes eines optischen Trackingsystem werden die folgenden Schritte durchgeführt:
- ein Kalibrierungsgerät, welches eine Trackingmarker-Konfiguration aufweist, wird den Erfassungsbereich des Trackingsystems gebracht;
- die Trackingmarker des Kalibrierungsgeräts werden vom Trackingsystem erfasst;
- die Anordnung der Trackingmarker wird verändert und/oder in einen Zustand gebracht, so dass das Trackingsystem ein Erfassungsproblem erfährt;
- die Anordnung der Trackingmarker, bei der das Erfassungsproblem besteht, wird vermessen bzw. wird ein Anordnungsbereich für die Trackingmarker vermessen, bei dem das Erfassungsproblem besteht; und
- es wird die Fehlkalibrierung des Trackingsystems festgestellt, wenn die vermessene Anordnung bzw. der Anordnungsbereich nicht einen vorbestimmten Wert hat oder nicht in einem vorbestimmten Wertebereich liegt.

Mit anderen Worten nutzt die vorliegende Erfindung die Tatsache aus, dass Trackingsysteme in manchen Fällen Probleme mit der richtigen Erfassung von Trackingmarkern haben. Erfindungsgemäß wird überprüft, ob die Probleme vorhanden sind, speziell wie stark diese Probleme sind bzw. waren und wie lange sie auftauchen, und aus diesen Informationen kann umgekehrt auf den Kalibrierungszustand des Trackingsystems geschlossen werden. Die entsprechenden Erfassungsprobleme treten nämlich dann auf, wenn die Kalibrierung sich verschlechtert. Die Erfindung macht also "aus der Not eine Tugend" indem sie gerade mögliche Fehlerfassungen dazu verwendet, einen Rückschluss auf die Kalibrierungsqualität eines Trackingsystems zu ziehen. Diese Rückschlüsse können qualitativer Arbeit (kalibriert/nicht kalibriert) sein, es ist aber auch möglich, quantitative Aussagen über den Umfang von Kalibrierungsfehlern zu machen.

Durch die Erfindung wird die Verifizierung der Kalibrierung eines Trackingsystems wesentlich vereinfacht, und sie kann durch den Verwender selbst oder einen Servicetechniker mit geringem Ausbildungsgrad durchgeführt werden. Die verwendeten Werkzeuge bzw. Kalibrierungsgeräte können zu niedrigen Kosten und in robuster Art für den Einsatz vor Ort bereitgestellt werden. Die Ermittlung des Kalibrierungszustandes hängt nicht von speziellen Ausführungen, Versionen, Herstellern oder Revisionen von stereoskopischen Trackingsystemen ab, da sie sich auf fundamentale physikalische Parameter bezieht, die bei jedem Trackingsystem vorhanden sind. Derselbe Test kann also bei verschiedensten Trackingsystemen angewendet werden.

Die Vermessung der Anordnung der Trackingmarker kann gemäß einer erfindungsgemäßen Ausführungsform durch ein Messmittel erfolgen, das dem Kalibrierungsgerät zugeordnet oder an ihm angebracht ist. Ferner können die Messergebnisse aus der Vermessung der Anordnung der Trackingmarker direkt am Messmittel oder am Kalibrierungsgerät ablesbar sein, wobei insbesondere am Kalibrierungsgerät Skalen oder Wertanzeigen angeordnet sind. Mit diesen Ausführungen lässt sich die Verifizierung sehr schnell, unmittelbar und direkt mit Hilfe des Kalibrierungsgeräts durchführen.

Bei einer Ausführungsvariante des erfindungsgemäßen Verfahrens werden bei der Vermessung der Anordnung der Trackingmarker lediglich einfache geometrische Verhältnisse wie Abstände oder Winkellagen gemessen. Einfache geometrische Verhältnisse sind dabei solche, die sich augenfällig, mit geringem Aufwand oder mit leicht beschaffbaren Mitteln messen lassen, wodurch sich die erfindungsgemäße Verifizierung auf einfachste Messvorgänge zurückführen lässt, die zu jedem Zeitpunkt und überall durchführbar sind.

Es besteht die Möglichkeit, bei dem Vergleich der Anordnung bzw. des Anordnungsbereiches mit dem vorbestimmten Wert bzw. dem Wertebereich relative Anordnungen der Trackingmarker zueinander oder relative Anordnungen der Trackingmarker-Konfiguration zueinander heranzuziehen. Ein Vorteil einer solchen Ausführung liegt darin, dass keine absoluten Punkte oder Winkel oder ähnliches im Erfassungsbereich des Trackingsystems eingestellt werden müssen, also keine Nullpunkte oder Nulllagen. Die Erfindung schließt jedoch die Vergleiche mit absoluten Werten, Wertebereichen oder Anordnungen nicht aus.

Ein Erfassungsproblem von Trackingsystemen, das bei der vorliegenden Erfindung Verwendung finden kann, ist das Auftreten von Ambiguitäten bei der Markererfassung, wobei die Anzahl erfasster potentieller Trackingmarker größer ist als die Anzahl tatsächlich im Erfassungsbereich vorhandene Trackingmarker. Dabei kann die Anordnung der Trackingmarker-Konfiguration zur Erzeugung des Auftretens der Ambiguitäten so eingestellt werden, dass die Trackingmarker sich im Wesentlichen in der Epipolarebene des Trackingsystems befinden. Eine Möglichkeit der Durchführung des Verfahrens besteht dann darin, die Trackingmarker-Konfiguration im Wesentlichen senkrecht zur Epipolarebene zu schwenken und mit dem Messmittel den Winkelbereich festzustellen, in dem die Ambiguitäten auftreten. Wenn der so festgestellte Winkel größer ist, als ein vorgegebener Winkelbereich kann die Fehlkalibrierung des Trackingsystems festgestellt werden. Je nachdem, wie viel größer der festgestellte Winkelbereich ist, kann auch eine quantitative Aussage über die Fehlkalibrierung getroffen werden.

Ein anderes Erfassungsproblem, das im Zuge der Durchführung der vorliegenden Erfindung herangezogen werden kann, ist das Auftreten einer ungenügenden Einzelmarker-Auflösung bei der Markererfassung. Dabei wird für zwei tatsächlich vorhandene Trackingmarker, die einen geringen Abstand voneinander haben, nur ein einziger Marker vom Trackingsystem erfasst.

In diesem Zusammenhang besteht die Möglichkeit das Kalibrierungsgerät für die Markererfassung an eine vorbestimmte Stelle im Erfassungsbereich des Trackingsystems, insbesondere in einen vorbestimmten Abstand zum Trackingsystem zu bringen, um vergleichbare bzw. gut aussagekräftige Werte zu erhalten. Wenn die oben zuletzt angesprochene Ausführungsform gewählt wird, kann die Anordnung der Trackingmarker zur Erzeugung des Auftretens der ungenügenden Einzelmarker-Auflösung so verändert werden, dass der Abstand der Trackingmarker zueinander verringert wird. Mit dem Messmittel kann der Trackingmarker-Abstand festgestellt werden, bei dem die Einzelauflösung ungenügend wird, und wenn dieser Abstand größer ist als ein vorgegebener Maximalabstand kann die Fehlkalibrierung des Trackingsystems festgestellt werden.

Eine erfindungsgemäße Vorrichtung zur Verifizierung des Kalibrierungszustands eines optischen Trackingsystems umfasst:
- ein Kalibrierungsgerät, welches eine Trackingmarker-Konfiguration aufweist;
- eine Trackingmarker-Bewegungseinrichtung, mittels welcher die Anordnung der Trackingmarker verändert und/oder in einen Zustand gebracht werden kann, so dass das Trackingsystem ein Erfassungsproblem erfährt;
- ein Messmittel, mit dem die Anordnung der Trackingmarker vermessen wird, bei der das Erfassungsproblem besteht bzw. ein Anordnungsbereich für die Trackingmarker vermessen wird, bei dem das Erfassungsproblem besteht; und
- eine Auswertungseinheit, bei welcher dann, wenn die vermessene Anordnung bzw. der Anordnungsbereich nicht einem vorbestimmten Wert hat oder nicht in einem vorbestimmten Wertebereich liegt, die Fehlkalibrierung des Trackingsystems festgestellt wird.

Die genannte Auswertungseinheit kann eine computergestützte, an das Trackingsystem und/oder das Kalibrierungsgerät angeschlossene Auswertungseinheit sein; sie kann separat mit einer solchen Funktion bereitgestellt werden oder auch in ein bereits vor Ort vorhandenes System integriert werden, beispielsweise in ein medizintechnisches Navigationssystem.

Das Messmittel kann dem Kalibrierungsgerät zugeordnet oder an ihm angebracht sein und ferner eine Anzeige aufweisen, auf der Messergebnisse aus der Vermessung der Anordnung der Trackingmarker ablesbar sind, wobei insbesondere am Kalibrierungsgerät Skalen oder Wertanzeigen angeordnet sind. Es besteht ferner natürlich auch die Möglichkeit, die Messergebnisse, eventuell zusammen mit dem Erfassungsergebnissen des Trackingsystems, auf einer separaten, der Auswertungseinheit oder einem Navigationssystem zugeordneten Anzeige auszugeben. Entsprechende Datenverbindungen können per Kabel oder kabellos bereitgestellt werden.

Das Messmittel ist bevorzugt eines, das einfache geometrische Verhältnisse wie Abstände oder Winkellagen misst, wobei sich die oben schon genannten Vorteile einstellen.

Wenn das Erfassungsproblem das Auftreten von Ambiguitäten ist, kann die Vorrichtung derart ausgestaltet werden, dass die Trackingmarker, insbesondere zwei Trackingmarker, im Abstand auf einer Halterung angebracht sind, die schwenkbar an dem Kalibrierungsgerät angeordnet ist. Dabei ist bevorzugt am Kalibrierungsgerät ferner eine Winkelskala zur Anzeige des Schwenkwinkels der Halterung oder der Marker-Konfiguration vorgesehen.

Wenn das Erfassungsproblem das Auftreten einer ungenügenden Einzelmarker-Auflösung bei der Markererfassung ist, kann die Vorrichtung so ausgeführt werden, dass die Trackingmarker, insbesondere zwei Trackingmarker, im Abstand auf einer Halterung angebracht sind, wobei eine Verstelleinrichtung für den Abstand der Trackingmarker zueinander vorgesehen ist. Gemäß einer Ausführungsvariante weist das Kalibrierungsgerät hierbei eine Schiene auf, und mindestens ein Trackingmarker ist verschiebbar auf der Schiene angeordnet, wobei der Abstand anhand des Verschiebungsweges an der Schiene gemessen wird.

Es ist noch festzustellen, dass das Auftreten von Ambiguitäten sowie eine ungenügende Einzelmarker-Auflösung nur beispielhaft für Erfassungsprobleme stehen, die im Zusammenhang mit der vorliegenden Erfindung genutzt werden können. Jegliche andere denkbare Erfassungsprobleme, die mindestens teilweise auf den Verlust der Kalibrierung eines Trackingsystems hindeuten können, sind erfindungsgemäß verwendbar.

Die Erfindung wird im Weiteren anhand von Ausführungsbeispielen näher erläutert. Sie kann alle hierin beschriebenen Merkmale einzeln sowie in jedweder sinnvollen Kombination umfassen. In den beiliegenden Zeichnungen zeigen:
- Figuren 1 und 2:: Darstellungen, die die grundlegende Arbeitsweise eines optischen, stereoskopischen Trackingsystems erläutern;
- Figur 3:: eine Darstellung die das Auftreten von Ambiguitäten bei der Trackingmarker-Erfassung erläutert;
- Figur 4:: eine erfindungsgemäße Vorrichtung in einer ersten Ausführungsform;
- Figuren 5 und 6:: Darstellungen, die eine Funktionsweise eines Trackingsystems in Verbindung mit auftretenden Strahlabweichungen erläutern;
- Figur 7:: eine zweite Ausführungsform einer erfindungsgemäßen Vorrichtung; und
- Figur 8: eine Darstellung, welche ein Minimaldistanz-Volumen im Trackingsystem-Erfassungsbereich aufzeigt.

Die vorliegende Erfindung, mit welcher der Kalibrierungszustand eines optischen, stereoskopischen Trackingsystems ermittelt werden kann, ohne die internen Kalibrierungsparameter (z.B. Kameraparameter) oder das zugrunde liegende Kalibrierungsmodell kennen zu müssen, wird im Weiteren näher erläutert. Vorab soll aber zum besseren Verständnis noch dargestellt werden, wie ein stereoskopisches, dreidimensionales Trackingsystem arbeitet.

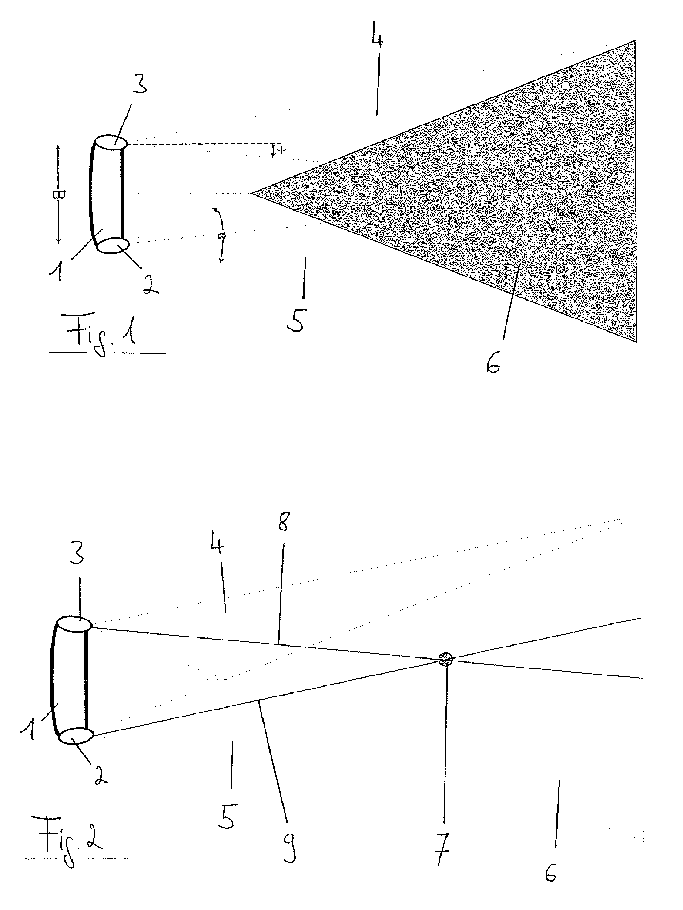

In den Figuren 1 und 2 trägt ein Trackingsystem das Bezugszeichen 1 und es weist zwei Kameras bzw. Sensoren 2, 3 auf. Diese beiden Kameras oder Bereichssensoren (typischerweise CCD oder CMOS-Sensoren) nehmen Bilder mit einem bestimmten Sichtwinkel auf. Der Abstand der beiden Kameras (Sensoren) 2, 3 wird als Basisabstand B bezeichnet. Der Winkel φ (Phi) beschreibt den Neigungswinkel, welcher es gestattet, die Überlappung der beiden Sichtfelder 4, 5 einzustellen, die den Erfassungsbereich 6 ergibt. Der Winkel α (Alpha) beschreibt den Sichtwinkel jedes Sensors bzw. jeder Kamera (optisches System). Bei vielen stereoskopischen Systemen, die für die medizinische bildgeführte Chirurgie verwendet werden liegt der Basisabstand B im Bereich von 150 mm bis 1000 mm, und der Winkel φ liegt im Bereich von 0 bis 20 Grad. Der Sichtwinkel α jedes Sensors liegt typischerweise im Bereich von 10 bis 80 Grad.

Die Kalibrierung des Systems definiert nunmehr die exakten Parameter der körperlichen Anordnung der beiden Sensoren zueinander (Abstand, Neigung, Rotation, Torsion ...). Diese Parameter werden oft als "externe Kalibrierungparameter" bezeichnet, da sie die externe Relation der Sensoren zueinander definieren. Zusätzlich zu diesen externen Parametern werden noch "interne Parameter" jeder Sensoranordnung in die Kalibrierung mit einbezogen (beispielsweise die Brennweite, die Linsenkrümmung, die Ausrichtung, der Mittelpunkt ... ).

Die internen Parameter können sehr präzise eingestellt werden und durch ein stabiles Hardware-Design stabil gehalten werden. Die externen Parameter unterliegen aber durch spezielle Beanspruchungen oftmals starken Veränderungen, beispielsweise bei Temperaturänderungen, mechanischen Stößen, Feuchtigkeitsänderungen, Materialermüdung, Materialausdehnung aufgrund der Schwerkraft (abhängig von der Aufstellung des Systems) und anderen Umgebungseinflüssen. Deshalb wird bei der Herstellung von vorkalibrierten Trackingsystem versucht, durch Konstruktionsmaßnahmen die Veränderung der externen Parameter gering zu halten, um so eine spezifizierte Genauigkeit über eine längere Zeitspanne gewährleisten zu können.

Kritische Bedingungen entstehen beispielsweise beim Transport des Systems vom Hersteller zum Endverbraucher oder bei der Handhabung (Fallen, Stoßen) durch den Endverbraucher. Für diesen ist es dann nicht einfach, festzustellen, ob ein solcher Vorfall das System beschädigt oder die Spezifikationen beeinflusst hat.

Die geforderte Genauigkeit für 3D-Markerpositionen erreicht für viele medizinische Anwendungen Werte von 0,1 bis 1 mm in großen Erfassungsbereichen (verglichen mit der Größe des stereoskopischen Systems bzw. dessen Basisabstand B). Typischerweise liegt das kalibrierte Volumen im Bereich von 1 bis 5 m bei einem Basisabstand von 50 bis 80 cm. Daraus resultieren maximal zulässige Änderungen der externen Kalibrierungsparameter in der Größenordnung von nur wenigen Mikrometern, wenigen Milli-Grad oder sogar noch weniger. Ein kalibriertes System erfasst einen Marker im Erfassungsbereich 6 (Figur 2), indem die dreidimensionale Position berechnet wird aus der Bildposition des Markers 7 auf jedem Sensor 2, 3 (Sichtstrahl 9, 8) und aus der bekannten (kalibrierten) Relation der Sensoren 2, 3 zueinander (Triangulation). Idealerweise schneiden sich die beiden Sichtstrahlen 8, 9 für den Marker 7 an exakt einem Punkt, und sie geben so die wahre Markerposition wieder. Aufgrund von Toleranzen, Bildverarbeitungs-Artefakten und anderen Unsicherheiten muss eine gewisse Toleranz bei der Vorhersage der Markerposition gestattet werden.

Wenn, wie in Figur 2 gezeigt ist, nur ein Marker erfasst werden soll, hat dies den Vorteil, dass die Strahlen 8 und 9 nur einen Kreuzungspunkt haben, was die Triangulation vereinfacht. Bei tatsächlichen Anwendungen würde eine einzige, dreidimensionale Markerposition es aber nicht gestatten, die Ausrichtung eines Objekts zu bestimmen oder ein medizinisches Instrument zu navigieren. Deshalb müssen mehrere Marker detektiert werden und die relativen Abstände gestatten es, sechsdimensionale Informationen für starre Körper oder dreidimensionale Abstände der Marker und Instrumente relativ zueinander zu ermitteln.

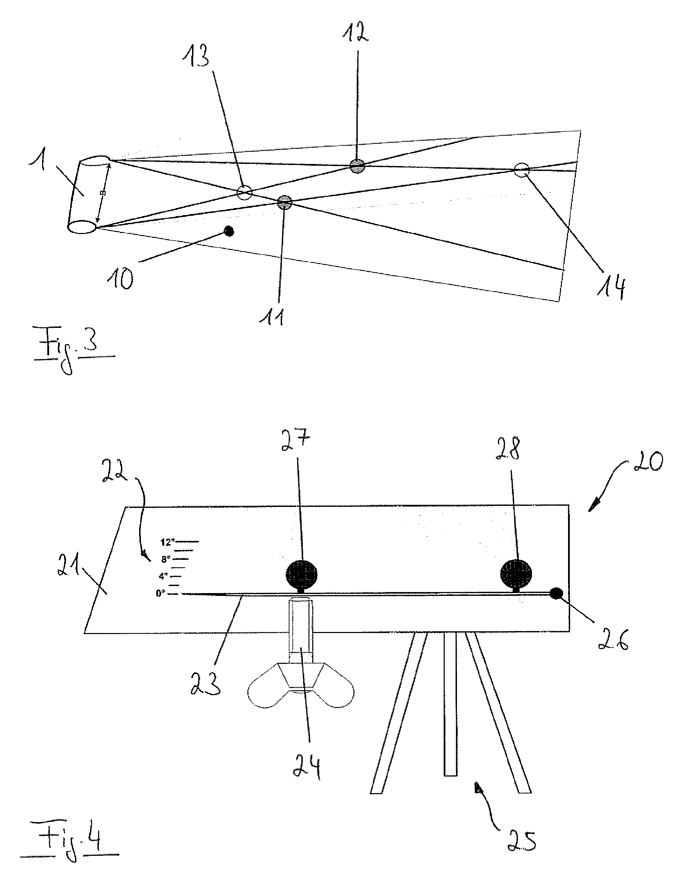

Bei vielen praktischen Anwendungsfällen entsteht tatsächlich nur ein Kreuzungspunkt für zwei Sehstrahlen im dreidimensionalen Erfassungsfeld des Trackingsystems, wodurch dann die Markerpositionen ermittelt werden können. Wenn die Marker aber innerhalb einer Ebene parallel zur Basis B angeordnet sind (Epipolarebene), würden mehrere Marker in dieser Ebene Ambiguitäten hervorrufen, da mehrere mögliche Kreuzungspunkte auftreten. Ein solcher Fall ist in der Figur 3 dargestellt, in welcher die Epipolarebene mit dem Bezugszeichen 10 angedeutet ist. In dieser Epipolarebene liegen zwei reale Marker 11, 12, aber der Triangulationsprozess würde vier potentielle Marker ergeben, nämlich zwei zusätzliche, nicht real vorhandene Marker 13 und 14. Die Sehstrahlen aus jeder Kamera schneiden sich nämlich insgesamt viermal. Wenn die Anzahl der Marker in einer Ebene parallel zur Basis B n beträgt, steigt die Anzahl der Ambiguitäten um n².

Das beschriebene Problem mit den Ambiguitäten wird, wenn möglich, durch einen Vergleich der Markergrößen, Markerhelligkeiten oder anderer Eigenschaften gelöst, um die Navigation durchführen zu können. Dazu ist jedoch ein relativ großer Bildverarbeitungs- und Rechenaufwand notwendig.

Die vorliegende Erfindung nutzt unter anderem gerade den Umstand, dass solche Ambiguitäten-Probleme auftreten können für die Verifizierung der Trackingsystem-Kalibrierung. Es wird also die "Fähigkeit" des Trackingsystems, solche Ambiguitäten zu produzieren, als Qualitätskriterium für das Trackingsystem verwendet. Ein gut kalibriertes System würde Ambiguitäten nur in einem sehr geringen Bereich erzeugen, nämlich wenn die Trackingmarker sehr genau parallel zur Basis B liegen. Die Verwendung eines einfachen Werkzeuges (beispielsweise eines Pointer-Werkzeuges mit zwei Trackingmarkern) und das Rotieren des Werkzeugs senkrecht zur Epipolarebene kann einen Neigungsbereich für die beiden Marker ergeben, in dem Ambiguitäten erzeugt werden. Ein gut kalibriertes System hätte nur einen sehr geringen solchen Bereich, während ein wenig gut kalibriertes bzw. ungenaues System einen größeren Bereich aufweisen würde. Damit kann ein Ambiguitäten-Winklel (β) definiert werden, der als Qualitätskriterium zum Testen von Trackingsystemen vor Ort beim Benutzer verwendbar ist.

Wie oben schon angesprochen, kann der Ambiguitäten-Winkel (β) mit einem einfachen Werkzeug ermittelt werden, das zwei Trackingmarker aufweist. Die absolute Winkelanordnung des Werkzeugs bzw. der Trackingmarker spielt auch keine unmittelbare Rolle bei der Bestimmung des Winkels, weil er basierend auf der Fragestellung ermittelt werden kann, was der maximale Winkelbereich ist, in dem die beiden Marker Ambiguitäten erzeugen. Idealerweise würde das System nur eine sehr geringe Neigung (einen geringen Ambiguitäten-Winkel) haben. Abhängig von den internen Systemparametern und dem zugrunde liegenden Kalibrierungsmodell kann der Winkel (β) im Erfassungsvolumen variieren oder nicht. Bei einem bekannten System können Spezifikationen für den Winkel (β) gegeben oder durch den Verwender ermittelt werden und Änderungen sind leicht mittels des einfachen Werkzeugs erfassbar.

Eine Ausführungsform eines solchen Kalibrierungsgeräts (Gerät zur Prüfung der Kalibrierung) ist in der Figur 4 gezeigt. Das Kalibrierungsgerät 20 aus Figur 4 steht auf drei Standbeinen 25 und es weist eine Rückplatte 21 auf, auf die eine Winkelskala aufgebracht ist. Am Schwenkpunkt 26 ist eine Zeigernadel 23 gelagert, und auf der Zeigernadel sitzen im Abstand zwei Trackingmarker 27 und 28. Die Neigung der Nadel 23 kann mit der Stellschraube 24 eingestellt werden.

Mit dieser einfachen Konfiguration lässt sich nun die Nadel 23 im Winkel verändern und über die Skala 22 können der minimale und der maximale Winkel ausgelesen werden, bei denen Ambiguitäten auftreten. Weil eine einfache Subtraktion dann den Ambiguitäten-Winkel (β) ergibt, muss bei einem solchen Ansatz noch nicht einmal der anfängliche Winkel zur Epipolarebene bekannt sein. Es genügt wenn hier keine absoluten Raumwinkelwerte ermittelt werden, sondern nur relative Werte, die dann über den Ambiguitäten-Winkel (β) ausreichend Auskunft geben. Andere Ausführungsformen sind möglich; sie könnten ein Kalibrierunggerät umfassen, das eine oder zwei Laserlinien erzeugt, die mit der Basis B des Trackingsystems ausgerichtet werden können. Der Winkel (β) kann durch das Drehen des Gerät mit dem zweiten Laser gemessen werden, sowie durch das Bestimmen der Winkelrelation der beiden Laserlinien.

Ein großer Vorteil dieser Kalibrierungs-Evaluierung liegt darin, dass sie vollständig unabhängig von der Art des Trackingsystems ist; man muss nur prüfen, ob das Trackingsystem mehr als zwei erwartete Markerpositionen übermittelt und dann den Bereich definieren, in welchem dies möglich ist, bzw. möglich sein darf.

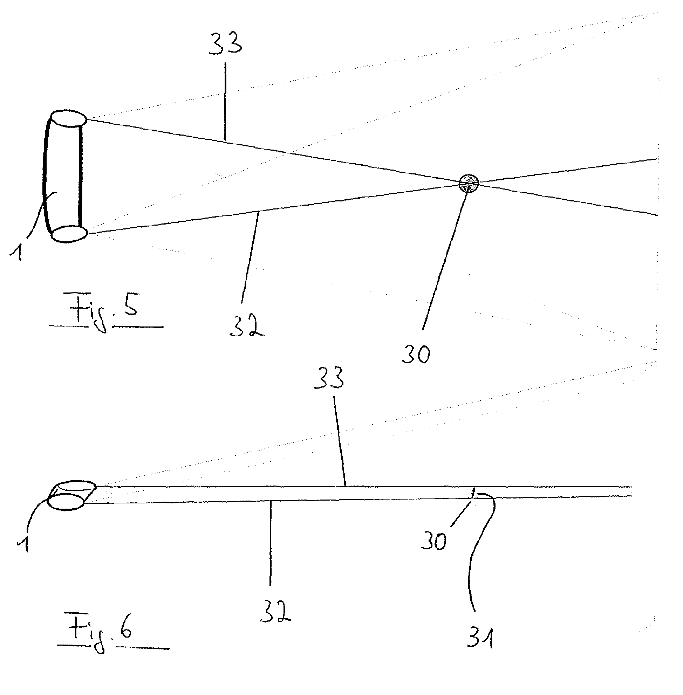

Eine weitere Ausführungsform der vorliegenden Erfindung arbeitet mit Strahlabweichungen und der sogenannten Einzelmarker-Auflösung. Oben ist schon angesprochen worden, dass die beiden Sehstrahlen sich idealerweise an exakt einem Punkt schneiden, der die Markerposition angibt, aber aufgrund von Toleranzen, Bildverarbeitungsartefakten, Störungen und anderen Unsicherheiten eine gewisse Toleranz bei der Marker-Positionserfassung gestattet werden muss. Diese Toleranzspanne wird als "Strahlabweichung" bezeichnet. Es ist zu bemerken, dass es sich hierbei nicht nur um einen zweidimensionalen Abstand sondern um dreidimensionalen Abstand handelt, nämlich den kleinsten räumlichen Abstand zwischen den beiden Sehstrahlen. Die Strahlabweichung jedes Markers ist im Grunde ein Vektor mit einer Richtung (kleinster Abstand des einen Strahls zu dem anderen). Aufgrund der geometrischen Eigenschaften des Triangulations-Systems und dem zugrunde liegenden Projektionsmodell ist die Definition einer Strahlabweichung sinnvoll und auch typischerweise in der Literatur zu finden. Die Figuren 5 und 6 zeigen in einer Ansicht von oben (Figur 5) und in einer seitlichen Ansicht (Figur 6) wie die beiden Sehstrahlen 32, 33 sich toleranzbehaftet an einem Marker 30 schneiden. In der Ansicht von oben in Figur 5 sieht es so aus, als würden sich die beiden Strahlen 32, 33 direkt auf dem Marker schneiden, jedoch zeigt die seitliche Ansicht der Figur 6, dass die beiden Strahlen etwas räumlich zueinander versetzt (windschief) sind, und der geringste räumliche Abstand der beiden Strahlen ist mit dem Bezugszeichen 31 gekennzeichnet.

Der Strahlabweichungs-Abstand oder Strahlabweichungs-Winkel ist naturgemäß ein nützlicher interner Parameter zur Bestätigung der Qualität der Markererfassung, und ergibt wiederum ein Qualitätskriterium für die Kalibrierung. Wenn der Strahlabweichungs-Abstand zu groß ist, kann ein Ausrichtungsfehler des Sensorsystems angenommen werden, und die Gesamtkalibrierung des Systems ist sehr wahrscheinlich verloren. Typischerweise wird für Trackingsysteme ein Maximalwert für die Strahlabweichung an einem einzelnen Markerpunkt definiert und Markerpositionen die nicht in diesen Bereich fallen, werden nicht verwendet. Da eine Fehlausrichtung der Sensoren typischerweise nur in einem erhöhten Strahlabweichungs-Winkel resultiert, ist ein typischer Effekt solcher de-kalibrierten Systeme, dass Markerpositionen nicht länger über das gesamte Erfassungsvolumen erfasst werden können, sondern typischerweise bei kleineren Abständen vom Trackingsystemen oder in mittleren Bereichen des Tracking-Volumens. Deshalb kann der Verwender in einfacher Weise grob die Leistungsfähigkeit des Systems feststellen, indem er prüft, ob Marker am nahen oder fernen Ende des kalibrierten Volumens noch getrackt werden können.

Wenn die Strahlabweichung noch in einem spezifizierten Bereich liegt, ist sie nicht zur Kalibrierungsprüfung geeignet, und dies gilt auch dann, wenn für handelsübliche Systeme keine Ermittlung der Strahlabweichung möglich ist. Für diesen Fall erweitert die vorliegende Erfindung das Szenario der Strahlabweichung durch die Verwendung einer Markerkonfiguration, also durch die Verwendung von mehr als einem Marker in spezieller Anordnung, und sie greift so auf einen speziellen Parameter stereoskopischer Trackingsysteme zurück, der als "Einzelmarker-Auflösung" bezeichnet wird. Dieser Begriff definiert die Fähigkeit eines Trackingsystems zwischen zwei einzelnen Marker zu unterscheiden, die nahe beieinander stehen. Wenn der Abstand der beiden Marker geringer wird, wird das System an einem bestimmten Punkt nicht länger dazu in der Lage sein, zwischen den Markern zu unterscheiden. Dies resultiert entweder darin, dass nur ein Marker gemeldet wird, oder dass aufgrund interner Kriterien wie erwartete Markerform oder einem Minimalabstands-Kriterium, kein Marker gemeldet wird.

Basierend auf der Tatsache, dass die Markerauflösung nicht höher sein kann als die Strahlabweichung, die im System gestattet wird, ist zu erwarten, dass der geringste Markerabstand zweier Marker nicht über die räumliche Anordnung der beiden Marker konstant ist. Er wird sich mit dem Abstand von der Sensoreinheit ändern.

Für einen vorgegebenen räumlichen Abstand vom Trackingsystem kann aber der Minimalabstand der beiden Marker so erfasst werden, dass er eine wertvolle Information über die Genauigkeit des Systems gibt und es dem Verwender gestattet, die Kalibrierung zu evaluieren.

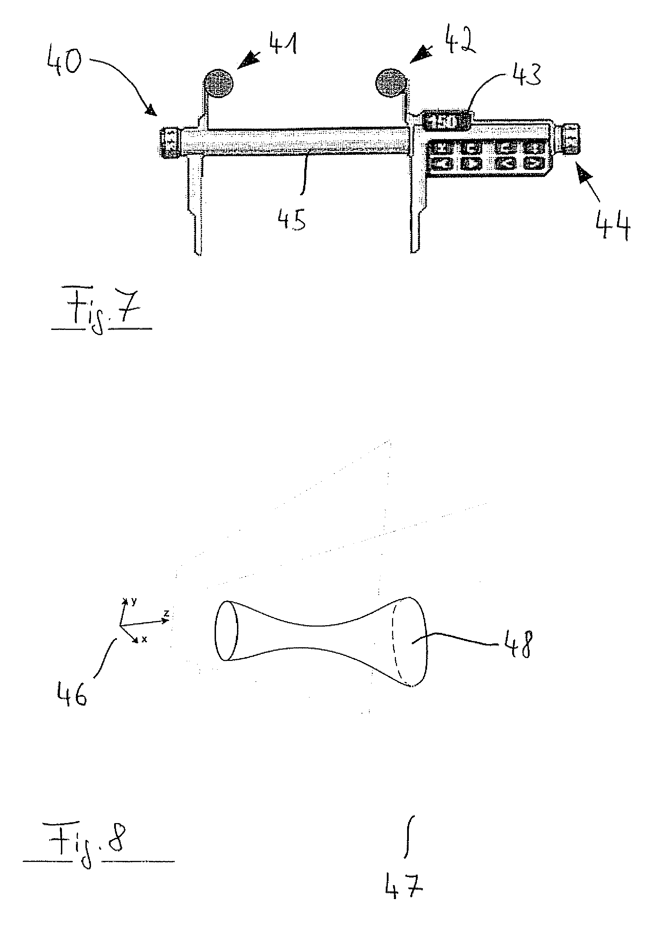

Als Kalibrierungsgerät (Gerät zum Prüfen der Kalibrierung) kann hierbei beispielsweise ein Gerät verwendet werden, das in Figur 7 dargestellt ist und insgesamt das Bezugszeichen 40 trägt. Das Gerät 40 ist grundsätzlich wie eine Schieblehre mit einer Schiene 45, einer elektronischen Abstandsanzeige 43 und manuellen/elektrischen Verstelleinrichtungen 44 ausgestaltet. Es trägt die beiden Trackingmarker 41 und 42, und der Abstand zwischen den beiden Markern (Zentrumsabstand) wird auf der Anzeige 43 angezeigt. Man kann also auf dem Kalibrierungsgerät 40 genau ablesen, wie weit die Marker 41 und 42 beabstandet sind und dann prüfen, ob das Trackingsystem die Marker bei einem bestimmten Abstand noch als einzelne Marker erkennt.

Grundsätzlich ist als Kalibrierungsgerät jedes Gerät verwendbar, das zwei Marker und eine Anzeige über den Abstand der zwei Marker umfasst.

Wenn man die zu erwartende Leistungsfähigkeit des Systems kennt, kann ein Benutzer einfach den Markerabstand am Gerät 40 auf den erforderlichen Minimalabstand einstellen und - geführt durch ein Computerprogramm, welches die Trackingsystem-Ausgabe auswertet - verifizieren, ob das System dazu in der Lage ist, zwei Marker in einem vorgegebenen Bereich im kalibrierten Volumen zu identifizieren. Wenn dies nicht der Fall ist, ist das System höchstwahrscheinlich dekalibriert. Ferner kann der Verwender die Größe des Kalibrierungsfehlers feststellen, indem er die Marker weiter auseinander bringt und dann die Messanzeige abliest, bei der zwei Marker gemeldet werden.

In der Figur 8 ist ein Trackingsystem-Erfassungsbereich 47 dargestellt, zusammen mit den Flächenkoordinaten X und Y sowie der Tiefenkoordinate Z. Wenn man annimmt, dass Trackingsysteme in der X- und Y-Richtung ähnliche oder gleiche Auflösungen haben, wäre zu erwarten, dass für den genannten Minimalabstand eine eliptische oder kreisförmige Form mit einem kleinsten Durchmesser entsteht, der typischerweise etwa im Zentrum (Z-Achse) des kalibrierten Volumens liegt. Die Figur 8 zeigt mit dem Bezugszeichen 48 eine typische Form eines Feldes für den minimalen Abstand.

Um noch mehr Informationen über den Kalibrierungszustand des Trackingsystems zu erhalten, kann die Position (speziell die Z-Position) für den minimalen Durchmesser im Trackingvolumen bestimmt werden. Wenn die Minimum-Position bekannt ist (durch Spezifizierung des Systems oder eine vorhergehende Bestimmung) kann leicht beurteilt werden, ob Veränderungen stattgefunden haben, welche die Genauigkeit beeinflussen können.

## Patentansprüche

1. Verfahren zur Verifizierung des Kalibrierungszustandes eines optischen Trackingsystems (1), bei dem
- ein Kalibrierungsgerät (20, 40), welches eine Trackingmarker-Konfiguration (27, 28; 41, 42) aufweist, in den Erfassungsbereich des Trackingsystems (1) gebracht wird;
- die Trackingmarker (27, 28; 41, 42) des Kalibrierungsgeräts (20, 40) vom Trackingsystem erfasst werden;
- die Anordnung der Trackingmarker (27, 28; 41, 42) verändert und/oder in einen Zustand gebracht wird, so dass das Trackingsystem (1) ein Erfassungsproblem erfährt;
- die Anordnung der Trackingmarker (27, 28; 41, 42) vermessen wird, bei der das Erfassungsproblem besteht bzw. ein Anordnungsbereich für die Trackingmarker (27, 28; 41, 42) vermessen wird, bei dem das Erfassungsproblem besteht; und bei dem
- wenn die vermessene Anordnung bzw. der Anordnungsbereich nicht einen vorbestimmten Wert hat oder nicht in einem vorbestimmten Wertebereich liegt, die Fehlkalibrierung des Trackingsystems (1) festgestellt wird.

2. Verfahren nach Anspruch 1, bei dem die Vermessung der Anordnung der Trackingmarker (27, 28; 41, 42) durch ein Messmittel erfolgt, das dem Kalibrierungsgerät (20, 40) zugeordnet oder an ihm angebracht ist.

3. Verfahren nach Anspruch 1 oder 2, bei dem die Messergebnisse aus der Vermessung der Anordnung der Trackingmarker (27, 28; 41, 42) direkt am Messmittel oder am Kalibrierungsgerät (20, 40) ablesbar sind, wobei insbesondere am Kalibrierungsgerät Skalen oder Wertanzeigen (22, 43) angeordnet sind.

4. Verfahren nach einem der Ansprüche 1 bis 3, bei dem bei der Vermessung der Anordnung der Trackingmarker (27, 28; 41, 42) lediglich einfache geometrische Verhältnisse wie Abstände oder Winkellagen gemessen werden.

5. Verfahren nach einem der Ansprüche 1 bis 4, bei dem bei dem Vergleich der Anordnung bzw. des Anordnungsbereichs mit dem vorbestimmten Wert bzw. dem Wertebereich relative Anordnungen der Trackingmarker (27, 28; 41, 42) zueinander oder relative Anordnungen der Trackingmarker-Konfiguration zueinander herangezogen werden.

6. Verfahren nach einem der Ansprüche 1 bis 5, bei dem das Erfassungsproblem das Auftreten von Ambiguitäten bei der Markererfassung umfasst, wobei die Anzahl erfasster potentieller Trackingmarker größer ist als die Anzahl tatsächlich im Erfassungsbereich vorhandener Trackingmarker (27, 28).

7. Verfahren nach Anspruch 6, bei dem die Anordnung der Trackingmarker-Konfiguration zur Erzeugung des Auftretens der Ambiguitäten so eingestellt wird, dass die Trackingmarker (27, 28) sich im Wesentlichen in der Epipolarebene des Trackingsystems (1) befinden.

8. Verfahren nach Anspruch 6 oder 7, bei dem die Trackingmarker-Konfiguration im Wesentlichen senkrecht zur Epipolarebene (10) geschwenkt wird und mit dem Messmittel der Winkelbereich festgestellt wird, in dem die Ambiguitäten auftreten, wobei dann, wenn der Winkelbereich größer ist als ein vorgegebener Winkelbereich, die Fehlkalibrierung des Trackingsystems (1) festgestellt wird.

9. Verfahren nach einem der Ansprüche 1 bis 8, bei dem das Erfassungsproblem das Auftreten einer ungenügenden Einzelmarker-Auflösung bei der Markererfassung umfasst, wobei für zwei tatsächlich vorhandene Trackingmarker (41, 42), die einen geringen Abstand voneinander haben, ein einziger Marker vom Trackingsystem (1) erfasst wird.

10. Verfahren nach Anspruch 9, bei dem das Kalibrierungsgerät (20, 40) für die Markererfassung an eine vorbestimmte Stelle im Erfassungsbereich des Trackingsystems (1), insbesondere in einen vorbestimmten Abstand zum Trackingsystem (1) gebracht wird.

11. Verfahren nach Anspruch 9 oder 10, bei dem die Anordnung der Trackingmarker (41, 42) zur Erzeugung des Auftretens der ungenügenden Einzelmarker-Auflösung so verändert wird, dass der Abstand der Trackingmarker zueinander verringert wird und mit dem Messmittel der Trackingmarker-Abstand festgestellt wird, bei dem die Einzelauflösung ungenügend wird, wobei dann, wenn der Abstand größer ist als ein vorgegebener Maximalabstand, die Fehlkalibrierung des Trackingsystems (1) festgestellt wird.

12. Vorrichtung zur Verifizierung des Kalibrierungszustandes eines optischen Trackingsystems (1), mit:
- einem Kalibrierungsgerät (20, 40), welches eine Trackingmarker-Konfiguration (27, 28; 41, 42) aufweist,
- einer Trackingmarker-Bewegungseinrichtung, mittels welcher die Anordnung der Trackingmarker (27, 28; 41, 42) verändert und/oder in einen Zustand gebracht werden kann, so dass das Trackingsystem (1) ein Erfassungsproblem erfährt;
- einem Messmittel (22, 43), mit dem die Anordnung der Trackingmarker (27, 28; 41, 42) vermessen wird, bei der das Erfassungsproblem besteht bzw. ein Anordnungsbereich für die Trackingmarker (27, 28; 41, 42) vermessen wird, bei dem das Erfassungsproblem besteht; und mit
- einer Auswertungseinheit, mit welcher dann, wenn die vermessene Anordnung bzw. der Anordnungsbereich nicht einen vorbestimmten Wert hat oder nicht in einem vorbestimmten Wertebereich liegt, die Fehlkalibrierung des Trackingsystems (1) festgestellt wird.

13. Vorrichtung nach Anspruch 12, **dadurch gekennzeichnet, dass** das Messmittel dem Kalibrierungsgerät (20, 40) zugeordnet oder an ihm angebracht ist.

14. Vorrichtung nach Anspruch 12 oder 13, **dadurch gekennzeichnet, dass** das Messmittel eine Anzeige aufweist, auf der die Messergebnisse aus der Vermessung der Anordnung der Trackingmarker (27, 28; 41, 42) ablesbar sind, wobei insbesondere am Kalibrierungsgerät (20, 40) Skalen oder Wertanzeigen angeordnet sind.

15. Vorrichtung nach einem der Ansprüche 12 bis 14, **dadurch gekennzeichnet, dass** das Messmittel eines ist, das einfache geometrische Verhältnisse wie Abstände oder Winkellagen misst.

16. Vorrichtung nach einem der Ansprüche 12 bis 15, **dadurch gekennzeichnet**, wobei das Erfassungsproblem das Auftreten von Ambiguitäten bei der Markererfassung umfasst, wobei die Anzahl erfasster potentieller Trackingmarker größer ist als die Anzahl tatsächlich im Erfassungsbereich vorhandener Trackingmarker (27, 28).

17. Vorrichtung nach Anspruch 16, **dadurch gekennzeichnet, dass** die Trackingmarker, insbesondere zwei Trackingmarker (27, 28), im Abstand auf einer Halterung angebracht sind, die schwenkbar an dem Kalibrierungsgerät (20) angeordnet ist, wobei am Kalibrierungsgerät (20) ferner eine Winkelskala zur Anzeige des Schwenkwinkels der Halterung oder der Marker-Konfiguration vorgesehen ist.

18. Vorrichtung nach einem der Ansprüche 12 bis 17, wobei das Erfassungsproblem das Auftreten einer ungenügenden Einzelmarker-Auflösung bei der Markererfassung umfasst, wobei für zwei tatsächlich vorhandene Trackingmarker, die einen geringen Abstand voneinander haben, ein einziger Marker als vorhanden vom Trackingsystem (1) erfasst wird.

19. Vorrichtung nach Anspruch 18, **dadurch gekennzeichnet, dass** die Trackingmarker, insbesondere zwei Trackingmarker (41, 42), im Abstand auf einer Halterung angebracht sind, wobei eine Verstelleinrichtung (44) für den Abstand der Trackingmarker (41, 42) zueinander vorgesehen ist.

20. Vorrichtung nach Anspruch 18 oder 19, **dadurch gekennzeichnet, dass** das Kalibrierungsgerät (40) eine Schiene (45) aufweist und mindestens ein Trackingmarker (42) auf der Schiene (45) verschiebbar angeordnet ist, wobei der Abstand anhand des Verschiebungswegs an der Schiene (45) gemessen wird.
